# EUROPEAN PATENT APPLICATION

(11) **EP 4 455 149 A1**
(43) Date of publication of application: **30.10.2024**
(21) Application number: 22911413.7
(22) Date of filing: 23.12.2022
(51) Int. Cl.: C07D 495/04

(54) **METHOD FOR PRODUCING BIOTIN DERIVATIVE**

(30) Priority: 24.12.2021 JP 2021211102
(71) Applicant: TOKUYAMA CORPORATION, Yamaguchi 745-8648 (JP)
(72) Inventor: MORI, Hiroyuki, Shunan-shi, Yamaguchi 745-8648 (JP); MATSUURA, Keisuke, Shunan-shi, Yamaguchi 745-8648 (JP)
(74) Representative: Hoffmann Eitle
(86) International application number: PCT/JP2022/047675
(87) International publication number: WO 2023/120712

(57) **Abstract**

The purpose of the present invention is to provide a method for producing a biotin derivative, which makes it possible to produce a biotin derivative at a high conversion rate even without using high-purity raw materials, and the present invention provides a method for producing a biotin derivative, including contacting, in a solvent containing 40 vol.% or more of a strong acid having an acid dissociation constant pKa of 1 or less, one or more derivatives selected from the group consisting of a hydroxybiotin derivative represented by formula (1) and a vinylbiotin derivative represented by formula (2) with a trialkylsilane compound to produce a biotin derivative represented by formula (3).

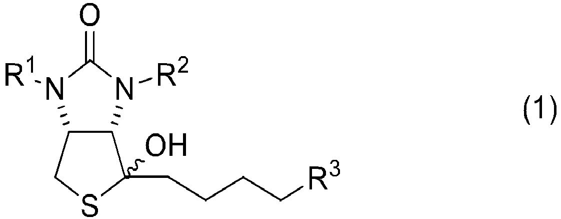

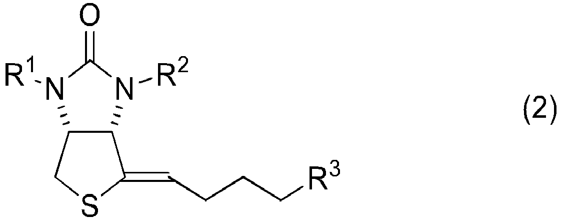

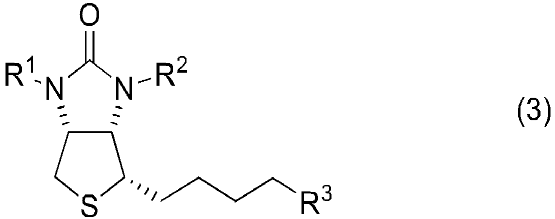

## Description

### TECHNICAL FIELD

The present invention relates to a novel method for producing a biotin derivative, and more particularly to a method for producing a biotin derivative through a reduction reaction of a hydroxybiotin derivative or a vinylbiotin derivative.

### BACKGROUND ART

Biotin is a useful compound that is used in various pharmaceuticals, food additives, feed additives, and the like. As a method for producing a biotin derivative, a method in which a vinylbiotin derivative is reduced to form a biotin derivative has been reported (see Non-Patent Document 1).

Non-Patent Document 1 discloses a method for producing a biotin derivative by reacting a hydroxybiotin derivative or vinylbiotin derivative having a benzyl ester group at a terminal of a side chain with triethylsilane as a reducing agent in a solvent containing trifluoroacetic acid and dichloromethane.

### PRIOR ART DOCUMENTS

### NON-PATENT DOCUMENT

Non-Patent Document 1: Tetrahedron Asymmetry, Vol. 19, 2008, pp. 1436-1443

### SUMMARY OF INVENTION

### TECHNICAL PROBLEM

However, according to the method described in Non-Patent Document 1, a high-purity hydroxybiotin derivative or vinylbiotin derivative that has been purified is used as a reaction substrate. Thus, this method necessitates carrying out a step of purifying the reaction substrate before use, and has room for improvement in terms of application in industrial production aimed at mass production.

### SOLUTION TO PROBLEM

As a result of intensive research to solve this issue, the present inventors surprisingly discovered that a biotin derivative can be synthesized at a high conversion rate by using a solvent containing a predetermined proportion or more of a strong acid having an acid dissociation constant pKa of 1 or less, even when a hydroxybiotin derivative and/or vinylbiotin derivative having a low purity is used as a reaction substrate, and the inventors thereby arrived at the present invention.

Thus, the present invention includes the following aspects.
[1] A method for producing a biotin derivative, the method including contacting, in a solvent containing 40 vol.% or more of a strong acid having an acid dissociation constant pKa of 1 or less,
   one or more derivatives selected from the group consisting of:
      a hydroxybiotin derivative represented by formula (1): 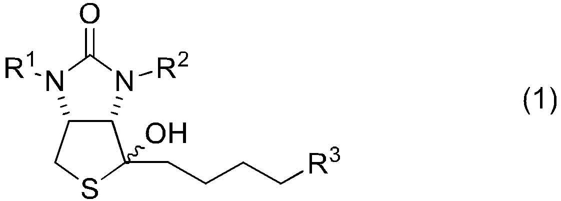 wherein
      R¹ and R² each independently represent a hydrogen atom, an alkyl group optionally having a substituent(s), an aralkyl group optionally having a substituent(s), or an aryl group optionally having a substituent(s),
      R³ represents a hydrogen atom, an alkyl group optionally having a substituent(s), a cyano group, or a monovalent group represented by the formula: -C(=O)OR⁴, and
      R⁴ represents a hydrogen atom, an alkyl group optionally having a substituent(s), an aralkyl group optionally having a substituent(s), or an aryl group optionally having a substituent(s); and
      a vinylbiotin derivative represented by formula (2): 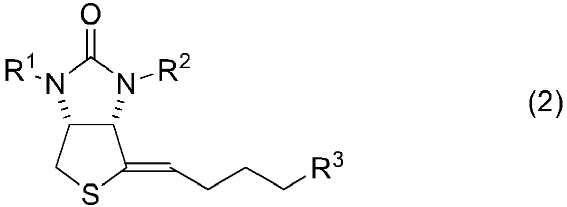
      wherein R¹, R² and R³ have the same definitions as R¹, R² and R³ in formula (1), respectively,
   with a trialkylsilane compound to produce a biotin derivative represented by formula (3): 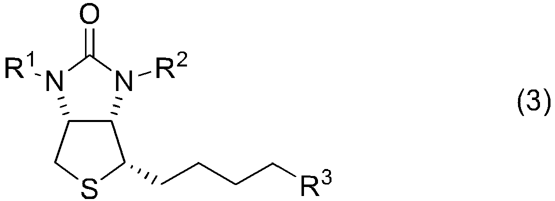
   wherein R¹, R² and R³ have the same definitions as R¹, R² and R³ in formula (1), respectively.
[2] The method for producing a biotin derivative according to [1],
   wherein the trialkylsilane compound is selected from the group consisting of trimethylsilane, triethylsilane, triisopropylsilane, and trihexylsilane.
[3] The method for producing a biotin derivative according to [1] or [2], wherein the one or more derivatives each have a purity of 95% or less as measured by liquid chromatography.
[4] The method for producing a biotin derivative according to [1] or [2], wherein 0.1 mL to 20 mL of the solvent is used per 1 g of the one or more derivatives.
[5] The method for producing a biotin derivative according to [3], wherein 0.1 mL to 20 mL of the solvent is used per 1 g of the one or more derivatives.

### EFFECTS OF INVENTION

The method for producing a biotin derivative according to the present invention makes it possible to produce a biotin derivative at a high conversion rate even without using a high-purity raw material.

### DETAILED DESCRIPTION OF INVENTION

The present invention relates to a method for producing a biotin derivative (3) represented by formula (3) by contacting one or more derivatives selected from the group consisting of a hydroxybiotin derivative (1) represented by formula (1) and a vinylbiotin derivative (2) represented by formula (2) with a trialkylsilane compound in a solvent containing 40 vol.% or more of a strong acid having an acid dissociation constant pKa of 1 or less. Hereinafter, details of the present invention will be described.

### [Descriptions of Terms]

Hereinafter, terms used in the present specification will be described. The following descriptions apply throughout the present specification unless otherwise specified. The expression "value A to value B" means the value A or more and the value B or less unless otherwise specified.

### Halogeno Group

Examples of the halogeno group include a fluoro group, a chloro group, a bromo group, and an iodo group.

### Alkyl Group

The carbon number of the alkyl group is, for example, 1 to 20, preferably 1 to 10, more preferably 1 to 8, more preferably 1 to 6, more preferably 1 to 4, more preferably 1 to 3, and more preferably 1 or 2. The alkyl group can be linear or branched. The carbon number of the linear alkyl group is 1 or more, and the carbon number of the branched alkyl group is 3 or more.

### Aryl Group

The aryl group is, for example, a monocyclic or polycyclic (e.g. bicyclic or tricyclic) aromatic hydrocarbon ring group. The carbon number of the aryl group is, for example, 3 to 22, preferably 3 to 20, more preferably 4 to 14, more preferably 6 to 14, and more preferably 6 to 10. The polycyclic ring is preferably a fused ring. Examples of the aryl group include a phenyl group and a naphthyl group. The aryl group is preferably a phenyl group.

### Aralkyl Group

The aralkyl group is an alkyl group having one or more aryl groups, and the alkyl group and the aryl group are the same as defined above. The number of the aryl group(s) contained in the aralkyl group is, for example, 1 to 3, preferably 1 or 2, and more preferably 1. Examples of the aralkyl group include a benzyl group, a phenylethyl group, a phenylpropyl group, a phenylbutyl group, and a naphthylmethyl group. The aryl group(s) contained in the aralkyl group is/are preferably a phenyl group. The aralkyl group is preferably a benzyl group.

### Alkoxy Group

The alkoxy group is a group represented by the formula: -O-alkyl group, and the alkyl group is the same as defined above.

### <Hydroxybiotin Derivative>

In the present invention, the hydroxybiotin derivative (1) is a compound represented by formula (1).

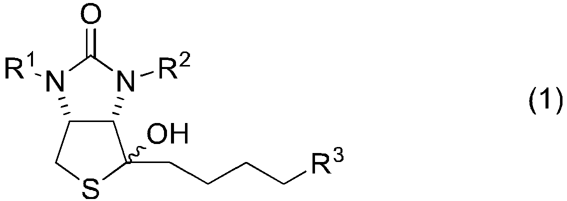

### (R¹ and R²)

In formula (1), R¹ and R² each independently represent a hydrogen atom, an alkyl group optionally having a substituent(s) (i.e., an alkyl group or an alkyl group having a substituent(s)), an aralkyl group optionally having a substituent(s) (i.e., an aralkyl group or an aralkyl group having a substituent(s)), or an aryl group optionally having a substituent(s) (i.e., an aryl group or an aryl group having a substituent(s)). R¹ and R² can be the same functional group, or can be different functional groups from each other.

Hereinafter, the alkyl group optionally having a substituent(s), the aralkyl group optionally having a substituent(s), and the aryl group optionally having a substituent(s) will be described.

### Alkyl group optionally having a substituent(s)

In one embodiment, R¹ and/or R² are/is an alkyl group optionally having a substituent(s). The alkyl group can be either linear or branched. The carbon number of the alkyl group is, for example, 1 to 20, preferably 1 to 10, more preferably 1 to 8, more preferably 1 to 6, more preferably 1 to 4, more preferably 1 to 3, more preferably 1 or 2, and particularly preferably 1. The alkyl group can have a substituent(s). Examples of the substituent(s) that the alkyl group can have include an aryl group having the carbon number of 3 to 22 (an aryl group having the carbon number of preferably 3 to 20, more preferably 4 to 14, more preferably 6 to 14, and more preferably 6 to 10), an alkoxy group having the carbon number of 1 to 6 (an alkoxy group having the carbon number of preferably 1 to 4, more preferably 1 to 3, and more preferably 1 or 2), and a halogeno group. The substituent(s) that the alkyl group can have is/are preferably an aryl group having the carbon number of 6 to 14, more preferably an aryl group having the carbon number of 6 to 10, and particularly preferably a phenyl group. When the alkyl group has a substituent(s), the number of the substituent(s) is preferably 1 to 5, more preferably 1 to 3, more preferably 1 or 2, and particularly preferably 1.

### Aralkyl group optionally having a substituent(s)

In one embodiment, R¹ and/or R² are/is an aralkyl group optionally having a substituent(s). The aralkyl group is preferably an aralkyl group having the carbon number of 7 to 11. Preferred examples of the aralkyl group include a benzyl group, a phenylethyl group, a phenylpropyl group, a phenylbutyl group, and a naphthylmethyl group. The aralkyl group can have a substituent(s). Examples of the substituent(s) that the aralkyl group can have include an alkoxy group having the carbon number of 1 to 6 (an alkoxy group having the carbon number of preferably 1 to 4, more preferably 1 to 3, and more preferably 1 or 2), a carboxyl group, and a halogeno group. When the aralkyl group has a substituent(s), the number of the substituent(s) is preferably 1 to 5, more preferably 1 to 3, more preferably 1 or 2, and particularly preferably 1.

### Aryl group optionally having a substituent(s)

In one embodiment, R¹ and/or R² are/is an aryl group optionally having a substituent(s). Examples of the aryl group include those that are monocyclic, bicyclic, or tricyclic. The aryl group is preferably an aryl group having the carbon number of 6 to 14, and particularly preferably a phenyl group. The aryl group can have a substituent(s). Examples of the substituent(s) that the aryl group can have include an alkoxy group having the carbon number of 1 to 6 (an alkoxy group having the carbon number of preferably 1 to 4, more preferably 1 to 3, and more preferably 1 or 2), a carboxyl group, and a halogeno group. When the aryl group has a substituent(s), the number of the substituent(s) is preferably 1 to 5, more preferably 1 to 3, more preferably 1 or 2, and particularly preferably 1.

Considering that R¹ and R² are ultimately removed in a de-protection step, R¹ and R² are each preferably an aralkyl group optionally having a substituent(s), more preferably an aralkyl group, and particularly preferably a benzyl group.

### (R³)

In formula (1), R³ is a hydrogen atom, an alkyl group optionally having a substituent(s), a cyano group, or a monovalent group represented by -C(=O)OR⁴, and R⁴ is a hydrogen atom, an alkyl group optionally having a substituent(s), an aralkyl group optionally having a substituent(s), or an aryl group optionally having a substituent(s). Among the alternatives of R³, the alkyl group optionally having a substituent(s), and among the alternatives of R⁴, the alkyl group optionally having a substituent(s), the aralkyl group optionally having a substituent(s), and the aryl group optionally having a substituent(s) are the same as defined above. The above descriptions about the alkyl group optionally having a substituent(s), the aralkyl group optionally having a substituent(s), and the aryl group optionally having a substituent(s) also apply to R³ and R⁴.

From the viewpoint of ease of conversion to biotin, R³ is preferably a cyano group, a carboxylic acid (R⁴ = a hydrogen atom), or an ester (R⁴ = an alkyl group optionally having a substituent(s)), more preferably a carboxylic acid (R⁴ = a hydrogen atom) or an ester (R⁴ = an alkyl group optionally having a substituent(s)), and particularly preferably an ester (R⁴ = an alkyl group).

### (Method for Producing Hydroxybiotin Derivative)

The hydroxybiotin derivative (1) of the present invention is not particularly limited, and one produced by a known method can be used. For example, a hydroxybiotin derivative produced by the method described in Non-Patent Document 1 and a purified product thereof can be used. Specifically, the hydroxybiotin derivative (1) can be produced according to the following reaction scheme.

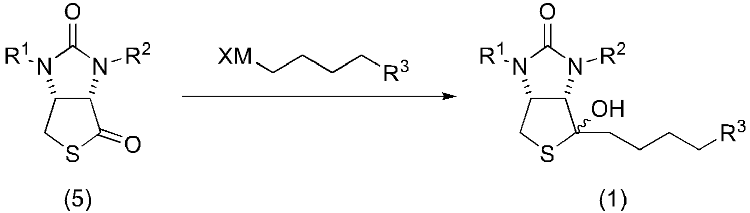

The compound represented by formula (5) is a thiolactone derivative and can be produced by a known method. In formula (5), R¹ and R² have the same definitions as R¹ and R² in formula (1), respectively. As shown in the above reaction scheme, the hydroxybiotin derivative (1) represented by formula (1) can be produced by reacting a thiolactone derivative (5) represented by formula (5) with an organometallic reagent represented by the formula: R³-(CH₂)₄-MX. In the organometallic reagent represented by the formula: R³-(CH₂)₄-MX, X is a halogen atom, M is a metal element selected from the group consisting of zinc and magnesium, and R³ has the same definition as R³ in formula (1).

The purity of the hydroxybiotin derivative (1) is not particularly limited. The hydroxybiotin derivative (1) can have a purity of, for example, 80.0 to 99.9% as measured by liquid chromatography. In the present invention, the purity as measured by liquid chromatography is a value in terms of area% measured by liquid chromatography. In the present invention, the purity as measured by liquid chromatography is preferably a purity as measured by high performance liquid chromatography (HPLC). The purity of the hydroxybiotin derivative (1) by HPLC is measured under the conditions described in the Examples below. In the present specification, the purity as measured by HPLC may be referred to as the "HPLC purity".

The method for producing a biotin derivative according to the present invention makes it possible to produce the biotin derivative (3) at a high conversion rate, even when the hydroxybiotin derivative (1) used as a raw material has a relatively low purity. Therefore, from the viewpoint of reducing the number of production steps, the hydroxybiotin derivative (1) used as a raw material is preferably not subjected to a purification step and is used in a crude form as a raw material. Specifically, even when the hydroxybiotin derivative (1) has a purity (preferably HPLC purity) of 95% or less as measured by liquid chromatography, the hydroxybiotin derivative (1) can be suitably used as a raw material in the method for producing a biotin derivative according to the present invention.

However, it is preferable that the purity of the hydroxybiotin derivative (1) used as a raw material is high since it is preferable that the purity of the finally obtained biotin derivative (3) is high. The purity of the crude form of the hydroxybiotin derivative (1) can be relatively increased by producing the hydroxybiotin derivative (1) by the following method, for example. When the purity of the hydroxybiotin derivative (1) used as a raw material is thus increased, this makes it possible to increase the purity of the biotin derivative (3) obtained by the method for producing a biotin derivative according to the present invention.

### (Preferred Method for Producing Hydroxybiotin Derivative)

Hereinafter, a preferred method for producing the hydroxybiotin derivative (1) will be described.

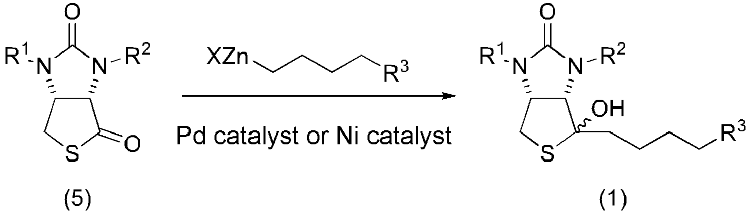

The hydroxybiotin derivative (1) can be produced by subjecting a thiolactone derivative (5) represented by formula (5) and an organozinc reagent (R³-(CH₂)₄-ZnX) for introducing a targeted side chain into the thiolactone derivative (5) to a Fukuyama coupling reaction in the presence of a palladium or nickel catalyst. In the organozinc reagent represented by the formula: R³-(CH₂)₄-ZnX, X represents a halogen atom. Examples of the halogen atom represented by X include a chlorine atom, a bromine atom, and an iodine atom. From the viewpoint of reactivity, the halogen atom is preferably a bromine atom or an iodine atom, and particularly preferably an iodine atom.

The organozinc reagent can be prepared by contacting a corresponding organohalide (R³-(CH₂)₄-X) with zinc in an organic solvent. The zinc used may be elemental zinc, and the form thereof is not limited. The zinc used is in the form of, for example, powder, turnings, and strips. The amount of the zinc used can be appropriately determined according to the type of the organohalide, and is, for example, 1 to 5 mol, and preferably 1 to 3 mol, per 1 mol of the organohalide.

Contacting the organohalide with the zinc is preferably carried out in the presence of a zinc activator. Examples of the zinc activator include bromine, iodine, 1,2-dibromoethane, and trimethylsilyl chloride. The amount of the zinc activator used is, for example, 0.01 to 1.5 mol, and preferably 0.2 to 0.8 mol, per 1 mol of the zinc.

Preparation of the organozinc reagent is preferably carried out in an organic solvent. Examples of the organic solvent include tetrahydrofuran, 2-methyltetrahydrofuran, toluene, cyclopentyl methyl ether, N,N-dimethylformamide, N,N-dimethylacetamide, tert-butyl methyl ether, diisopropyl ether, and diglyme. One of the above-exemplified organic solvents can be used alone, or a mixture of two or more thereof can be used.

The amount of the organic solvent used is not particularly limited, and is, for example, 0.5 to 20 mL, and preferably 1 to 10 mL, per 1 g of the zinc. When a mixture is used as the organic solvent, the amount of the organic solvent used is based on the total amount of the mixture. The temperature at which the contact between the organohalide and the zinc is carried out is, for example, 20 to 120°C, and preferably 30 to 100°C. The time for which the contact between the organohalide and the zinc is carried out is, for example, 0.1 to 10 hours, and preferably 1 to 5 hours.

The organozinc reagent is preferably prepared by the following method. First, an organic solvent and zinc are mixed, after which a zinc activator is added to activate the zinc. Next, an organohalide is added and mixed therewith, and thereby an organozinc reagent can be prepared. The resulting zinc reagent in a state of a solution can be directly used for producing the hydroxybiotin derivative (1), without further separating and purifying the resulting zinc reagent.

The hydroxybiotin derivative (1) can be obtained by subjecting the thiolactone derivative (5) and the organozinc reagent to a Fukuyama coupling reaction in the presence of a palladium or nickel catalyst. Examples of the palladium catalyst include palladium on carbon, palladium black, palladium acetate, palladium chloride, and bis(triphenylphosphine)palladium chloride. Examples of the nickel catalyst include nickel acetylacetonate and nickel chloride. From the viewpoint of reactivity, palladium acetate, palladium on carbon, and nickel acetylacetonate are preferred. The amount of the palladium or nickel catalyst used is not particularly limited, and is, for example, 0.001 to 0.5 mol, and preferably 0.005 to 0.05 mol, per 1 mol of the thiolactone derivative (5). The amount of the organozinc reagent used is not particularly limited, and is, for example, 1 to 5 mol, and preferably 1 to 3 mol, per 1 mol of the thiolactone derivative (5).

The organic solvent used in the synthesis reaction of the hydroxybiotin derivative (1) is not particularly limited as long as it does not inhibit the reaction, and thus the organic solvent can be used as necessary. Examples of the organic solvent include tetrahydrofuran, 2-methyltetrahydrofuran, toluene, cyclopentyl methyl ether, N,N-dimethylformamide, N,N-dimethylacetamide, tert-butyl methyl ether, diisopropyl ether, and diglyme. One of the above-exemplified organic solvents can be used alone, or a mixture of two or more thereof can be used. The amount of the organic solvent used is not particularly limited, and is for example, 3 to 30 mL, and preferably 5 to 20 mL, per 1 g of the thiolactone derivative (5). When a mixture is used as the organic solvent, the amount of the organic solvent used is based on the total amount of the mixture. When the organozinc reagent in a state of a solution is directly used, the total value (total amount) of the organozinc reagent along with any newly used organic solvent is adjusted to fall within the above-described range.

The Fukuyama coupling reaction can be carried out by mixing the palladium or nickel catalyst, the thiolactone derivative (5), the organozinc reagent, and the organic solvent. The order in which these components are mixed is not particularly limited. The Fukuyama coupling reaction can be carried out by stirring and mixing these components. In order to carry out the reaction effectively, the reaction is preferably carried out by the following method. It is preferable that first, the zinc reagent is prepared, a solution containing the zinc reagent is prepared, the thiolactone derivative (5) and optionally an organic solvent are added, and then the palladium or nickel catalyst is added. The temperature of the Fukuyama coupling reaction is not particularly limited, and from the viewpoint of the reaction conversion rate and the purity of the hydroxybiotin derivative (1), the temperature thereof is, for example, 20 to 100°C, and preferably 25 to 80°C. The reaction time is not particularly limited, and is, for example, 1 to 48 hours, and preferably 2 to 20 hours.

The hydroxybiotin derivative (1) can be produced by reaction under the above-described conditions. The method for isolating the hydroxybiotin derivative (1) from the reaction system is not particularly limited. Specifically, the hydroxybiotin derivative (1) can be isolated by dissolving the hydroxybiotin derivative (1) in a solvent that is hardly soluble in water, such as ethyl acetate, toluene, chloroform, or dichloromethane, and then subjecting the solution to washing with water, concentrating, drying, and the like. When a solvent hardly soluble in water is used as the solvent, the solution can be directly subjected to washing with water.

With respect to the hydroxybiotin derivative (1) obtained under the above-described conditions, the purity (preferably HPLC purity) as measured by liquid chromatography is not particularly limited. According to the above-described preferred production method, the hydroxybiotin derivative (1) having a purity (preferably HPLC purity) of 90.0 to 95.0% as measured by liquid chromatography can be obtained. The hydroxybiotin derivative (1) having such a purity can be suitably used as the reaction substrate of the present invention. A reaction liquid containing the hydroxybiotin derivative (1) can be directly converted into the vinylbiotin derivative (2).

### (Preferred Hydroxybiotin Derivative)

From the viewpoint of utility, preferred examples of the hydroxybiotin derivative (1) represented by formula (1) include a hydroxybiotin derivative (1A) represented by formula (1A) and a hydroxybiotin derivative (1B) represented by formula (1B). The hydroxybiotin derivative (1A) is a compound corresponding to the hydroxybiotin derivative (1) wherein R¹ and R² are both benzyl groups, and R³ is -CO₂Et (i.e., R⁴ is an ethyl group). The hydroxybiotin derivative (1B) is a compound corresponding to the hydroxybiotin derivative (1) wherein R¹ and R² are both benzyl groups, and R³ is -CO₂H (i.e., R⁴ is a hydrogen atom). In the formulas, "Bn" represents a benzyl group, and "Et" represents an ethyl group. Hereinafter, the same descriptions may be omitted.

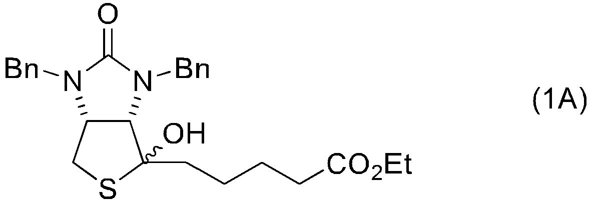

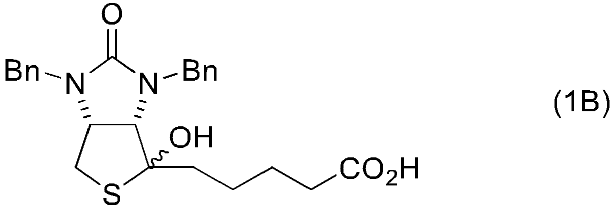

### <Vinylbiotin Derivative>

In the present invention, the vinylbiotin derivative (2) is a compound represented by formula (2).

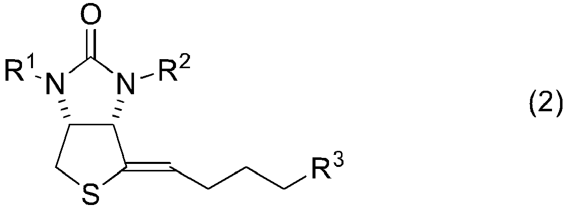

In the formula (2), R¹, R² and R³ have the same definitions as R¹, R² and R³ in formula (1), respectively.

The purity of the vinylbiotin derivative (2) is not particularly limited. The vinylbiotin derivative (2) can have a purity of, for example, 80.0 to 99.9% as measured by liquid chromatography.

The method for producing a biotin derivative according to the present invention makes it possible to produce the biotin derivative (3) at a high conversion rate, even when the vinylbiotin derivative (2) used as a raw material has a relatively low purity. Therefore, from the viewpoint of reducing the number of production steps, the vinylbiotin derivative (2) used as a raw material is preferably not subjected to a purification step and is used in a crude form as a raw material. Specifically, even when the vinylbiotin derivative (2) has a purity (preferably HPLC purity) of 95% or less as measured by liquid chromatography, the vinylbiotin derivative (2) can be suitably used as a raw material in the method for producing a biotin derivative according to the present invention.

However, it is preferable that the purity of the vinylbiotin derivative (2) used as a raw material is high since it is preferable that the purity of the finally obtained biotin derivative (3) is high. The purity of the crude form of the vinylbiotin derivative (2) can be relatively increased by producing the vinylbiotin derivative (2) by the following method, for example. When the purity of the vinylbiotin derivative (2) used as a raw material is thus increased, this makes it possible to increase the purity of the biotin derivative (3) obtained by the method for producing a biotin derivative according to the present invention.

The vinylbiotin derivative (2) of the present invention is not particularly limited, and can be produced by a known method. Specifically, as shown in the following reaction scheme, the vinylbiotin derivative (2) can be produced by subjecting the hydroxybiotin derivative (1) to a dehydration reaction.

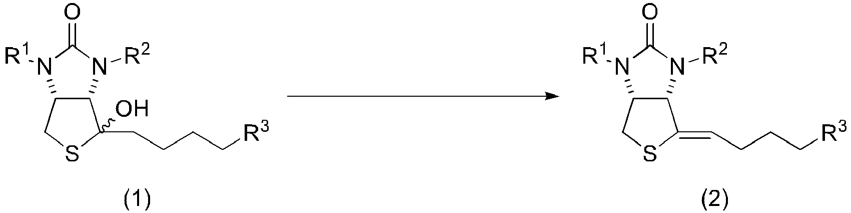

Examples of the method for dehydrating the hydroxybiotin derivative (1) include an acid treatment or a heat treatment. From the viewpoint of the purity of the resulting product, and the like, an acid treatment is preferable. The acid treatment includes contacting a solution of the hydroxybiotin derivative (1) with an acid catalyst. Examples of the acid catalyst include hydrochloric acid, sulfuric acid, p-toluenesulfonic acid, and mixtures thereof. The amount of the acid used is appropriately determined according to the type of the acid to be used, and is, for example, 1 to 1000 mol, and preferably 20 to 200 mol, per 1 mol of the hydroxybiotin derivative (1). The temperature at which the acid treatment is carried out is not particularly limited, and is, for example, 10 to 100°C, and preferably 20 to 70°C. The time for which the acid treatment is carried out is, for example, 0.1 to 10 hours, and preferably 1 to 7 hours.

The method for contacting the solution of the hydroxybiotin derivative (1) with the acid catalyst is not particularly limited. Examples of methods that can be used include a method in which the hydroxybiotin derivative (1), the acid catalyst, and a solvent capable of dissolving the hydroxybiotin derivative (1) are simultaneously mixed, and a method in which a solution in which the hydroxybiotin derivative (1) is dissolved (a reaction liquid obtained in the production of the hydroxybiotin derivative (1)) is prepared, and the acid catalyst is added to the solution and they are mixed. Among these methods, the method in which the acid catalyst is added to a reaction solution of the hydroxybiotin derivative (1) and they are mixed is preferably adopted from the viewpoint of operation ease.

The vinylbiotin derivative (2) can be easily produced by reaction under the above-described conditions. The method for isolating the vinylbiotin derivative (2) from the reaction system is not particularly limited. Specifically, the vinylbiotin derivative (2) can be isolated by dissolving the vinylbiotin derivative (2) in a solvent that is hardly soluble in water, such as ethyl acetate, toluene, chloroform, or dichloromethane, and then subjecting the solution to washing with water, concentrating, drying, and the like. When a solvent hardly soluble in water is used as the solvent, the solution can be directly subjected to washing with water.

With respect to the vinylbiotin derivative (2) obtained under the above-described conditions, the purity (preferably HPLC purity) as measured by liquid chromatography is not particularly limited. According to the above-described production method, the vinylbiotin derivative (2) having a purity (preferably HPLC purity) of 90.0 to 95.0% as measured by liquid chromatography can be obtained. The vinylbiotin derivative (2) having such a purity can be suitably used as the reaction substrate of the present invention.

### (Preferred Vinylbiotin Derivative)

From the viewpoint of utility, preferred examples of the vinylbiotin derivative (2) represented by formula (2) include a vinylbiotin derivative (2A) represented by formula (2A) and a vinylbiotin derivative (2B) represented by formula (2B). The vinylbiotin derivative (2A) is a compound obtained by a dehydration reaction of the hydroxybiotin derivative (1A) represented by formula (1A). The vinylbiotin derivative (2B) is a compound obtained by a dehydration reaction of the hydroxybiotin derivative (1B) represented by formula (1B).

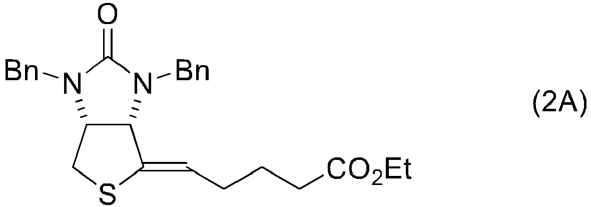

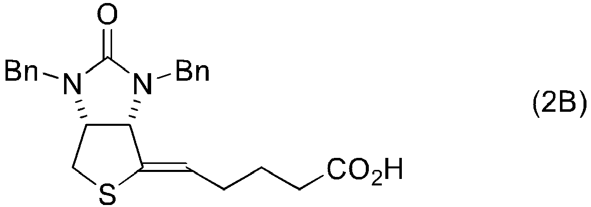

### <Reducing Agent>

In the present invention, the biotin derivative (3) is produced by contacting one or more derivatives selected from the group consisting of the hydroxybiotin derivative (1) and the vinylbiotin derivative (2) with a trialkylsilane compound as a reducing agent.

As the reducing agent used in the present invention, any of trialkylsilane compounds available as industrial raw materials or reagents can be used without any particular limitation.

The trialkylsilane compound is a compound represented by the formula: SiH-L¹(-L²)(-L³). L¹, L², and L³ are each independently an alkyl group. L¹, L², and L³ can be the same alkyl group or different alkyl groups. The alkyl group can be linear or branched. The carbon number of the alkyl group is preferably 1 to 10, more preferably 1 to 8, more preferably 1 to 7, more preferably 1 to 6, more preferably 1 to 4, and more preferably 1 to 3. The trialkylsilane compound is preferably one having the carbon number of 3 to 21. Preferred examples of the trialkylsilane compound used in the present invention include trimethylsilane, triethylsilane, triisopropylsilane, and trihexylsilane (e.g., tri-n-hexylsilane). Among these trialkylsilane compounds, triethylsilane and triisopropylsilane are particularly preferable since they can be expected to have a high reducing power.

The amount of the trialkylsilane compound used in the present invention is not particularly limited. From the viewpoint of achieving a desired reaction rate and avoiding complication of the post-treatment operation due to an excessively large amount of the trialkylsilane compound used, the amount of the trialkylsilane compound used is preferably 0.5 to 10.0 mol, and particularly preferably 1.0 to 5.0 mol, per 1 mol of the reaction substrate used in in the present invention (i.e., one or more derivatives selected from the group consisting of the hydroxybiotin derivative (1) and the vinylbiotin derivative (2)). In the present invention, when one type of derivative is selected as the reaction substrate, the amount of the reaction substrate means the amount of the one type of derivative, and when two or more types of derivatives are selected as the reaction substrate, the amount of the reaction substrate means the total amount of the two or more derivatives (the same applies throughout the present specification).

### <Solvent>

As the solvent of the present invention, a solvent containing 40 vol.% or more of a strong acid having an acid dissociation constant pKa of 1 or less is used.

The pKa used in the present invention refers to an acid dissociation constant (pKa) in an aqueous solution at 25°C. The "strong acid having a pKa of 1 or less" in the present invention is not limited to those which are liquid at room temperature, and even those which are solid at room temperature such as trichloroacetic acid can be used at a temperature equal to or higher than the melting point, or can be used by being dissolved in another solvent. Among these strong acids, it is preferable to use a strong acid that is a liquid at room temperature and also serves as a solvent. Examples of such a strong acid include trifluoroacetic acid (pKa = -0.3), methanesulfonic acid (pKa = -2.6), and trifluoromethanesulfonic acid (pKa = -14). Among these, trifluoroacetic acid and methanesulfonic acid are preferable, and trufluoroacetic acid is particularly preferable, from the viewpoint of reactivity.

The solvent of the present invention contains 40 vol.% or more of the above-described strong acid. In other words, the solvent of the present invention can contain 60 vol.% or less of a solvent(s) other than the above-described strong acid. From the viewpoint of the reaction rate, the content of the other solvent(s) is preferably 30 vol.% or less, and particularly preferably 0 vol.%. In other words, the solvent of the present invention preferably contains the above-described strong acid at an amount of 70 vol.% or more, and particularly preferably 100 vol.%. It should be noted that the expression "the solvent of the present invention contains the above-described strong acid at an amount of 100 vol.%" (that is, the content of a solvent(s) other than the above-described strong acid is 0 vol.%) does not completely exclude the inclusion of impurities that inevitably enter the solvent in addition to the above-described strong acid.

By increasing the proportion of the strong acid having a pKa of 1 or less in the reaction solvent of the present invention, the conversion rate to the biotin derivative (3) can be improved, and thus the reaction can be completed in a shorter amount of time. The other solvent(s) is/are not particularly limited as long as the other solvent(s) is/are stable in the presence of the strong acid and has/have no impact on the reaction according to the present invention. Specific examples of the other solvent(s) include dichloromethane, chloroform, and toluene.

In the present invention, the amount of the used solvent containing 40 vol.% or more of a strong acid having an acid dissociation constant pKa of 1 or less is not particularly limited. From the viewpoint of post-treatment of the reaction, and the like, the amount thereof is, for example, 0.1 to 20 mL, preferably 0.5 to 10 mL, and more preferably 3 mL to 7 mL, per 1 g of the reaction substrate of the present invention (i.e., one or more derivatives selected from the group consisting of the hydroxybiotin derivative (1) and the vinylbiotin derivative (2)). When a mixture containing the strong acid and a solvent(s) other than the strong acid is used as the solvent of the present invention, the amount of the solvent of the present invention is based on the total amount of the mixture.

### <Method for Producing Biotin Derivative>

The biotin derivative (3) can be produced by contacting the reaction substrate of the present invention (i.e., one or more derivatives selected from the group consisting of the hydroxybiotin derivative (1) and the vinylbiotin derivative (2)) with the trialkylsilane compound in the solvent containing 40 vol.% or more of a strong acid having an acid dissociation constant pKa of 1 or less. In this case, the components can be mixed so as to be sufficiently contacted with each other. The method according to the present invention can be carried out at atmospheric pressure, reduced pressure, or elevated pressure. The method according to the present invention can be carried out not only in the presence of oxygen such as in oxygen or in air, but also in an atmosphere of an inert gas such as nitrogen, argon, or carbon dioxide. The method of mixing the components is not particularly limited. For example, all of the components can be simultaneously inserted into a reaction device and mixed. Alternatively, one component can be mixed in advance, after which the remaining components can be added and mixed. Each component can be diluted with a solvent and supplied to the reaction device or the like. In order to further reduce the generation of byproducts and increase the purity of the biotin derivative (3), it is preferable to, in an inert gas atmosphere, mix and stir one or more derivatives selected from the group consisting of the hydroxybiotin derivative (1) and the vinylbiotin derivative (2) with the strong acid having a pKa of 1 or less or a solvent containing the strong acid having a pKa of 1 or less, and then add to the resultant mixture the trialkylsilane compound optionally diluted with a solvent, and then stir (mix) the resultant mixture.

In the present invention, the reaction temperature (temperature in the reaction system after all the components have been mixed) is not particularly limited. The reaction can be usually carried out at a temperature of 0 to 100°C. In this temperature range, the reaction is particularly preferably carried out at a temperature of 10 to 70°C from the viewpoint of the reaction rate and the purity of the biotin derivative (3) to be obtained. When the reaction is carried out within this temperature range, the reaction substrate can be efficiently converted to the biotin derivative (3) in a short amount of time. The reaction time is not limited and is appropriately determined while confirming the reaction conversion rate described in the following Examples. When the above-described reaction conditions are used, the reaction time is 1 to 72 hours, and preferably 1 to 24 hours. The term "reaction time" as used herein refers to the time for which the reaction substrate of the present invention, the solvent containing a strong acid having a pKa of 1 or less, and the trialkylsilane compound are mixed at the set reaction temperature.

The reaction liquid obtained in the present invention can be appropriately subjected to a post-treatment. Specifically, a crude product of the biotin derivative (3) can be collected by removing the solvent from the reaction liquid by distillation under reduced pressure or the like.

### <Biotin Derivative>

The biotin derivative (3) obtained in the present invention is a compound represented by formula (3).

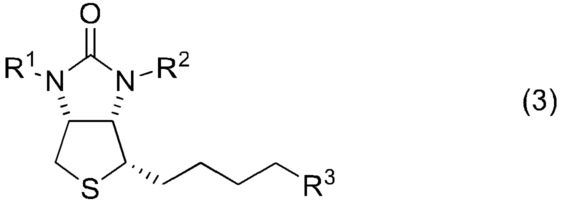

In formula (3), R¹, R² and R³ have the same definitions as R¹, R² and R³ in formula (1), respectively.

### (Preferred Biotin Derivative)

When a preferred raw material compound, i.e., the hydroxybiotin derivative (1A) or the vinylbiotin derivative (2A), or the hydroxybiotin derivative (1B) or the vinylbiotin derivative (2B) is used as a substrate, a biotin derivative represented by formula (3A) or (3B) can be obtained as the biotin derivative (3) represented by formula (3). The biotin derivative (3A) is a compound corresponding to the biotin derivative (3) wherein R¹ and R² are both benzyl groups, and R³ is -CO₂Et (i.e., R⁴ is an ethyl group). The biotin derivative (3B) is a compound corresponding to the biotin derivative (3) wherein R¹ and R² are both benzyl groups, and R³ is - CO₂H (i.e., R⁴ is a hydrogen atom), and can be used as the below-described biotin derivative (4).

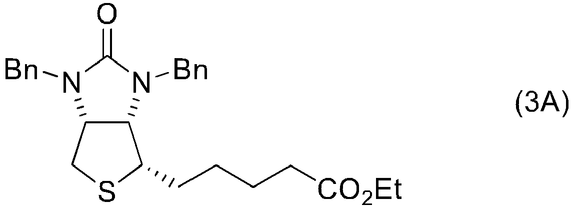

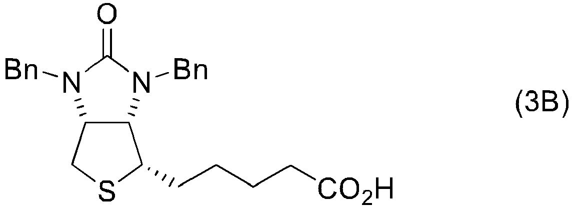

Biotin can be easily produced through a step of carrying out a de-protection treatment to remove the benzyl groups corresponding to R¹ and R² from the biotin derivative (3B).

In particular, the biotin derivative (3) wherein R³ is -CO₂R^{4'} (where R^{4'} is an alkyl group optionally having a substituent(s), an aralkyl group optionally having a substituent(s), or an aryl group optionally having a substituent(s)) can be easily converted into a biotin derivative (4) represented by formula (4) by subjecting the biotin derivative to an alkaline hydrolysis reaction. R^{4'} has the same definition as R⁴ except the case where R⁴ is a hydrogen atom. The above descriptions about the alkyl group optionally having a substituent(s), the aralkyl group optionally having a substituent(s), and the aryl group optionally having a substituent(s) in R⁴ also apply to R^{4'}. The biotin derivative (4) is useful as a biotin precursor, and biotin can be easily produced from the biotin derivative (4). Specifically, biotin can be easily produced through a step of carrying out a de-protection treatment to remove the functional groups represented by R¹ and R² from the biotin derivative (4). In the present specification, the biotin derivative (4) may be referred to as the "biotin precursor (4)".

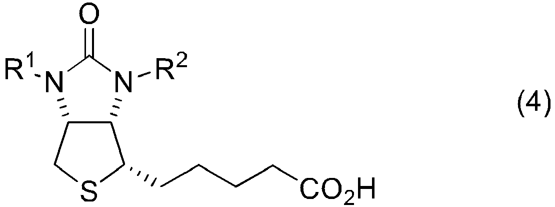

In particular, when the biotin derivative (3) wherein R³ is a cyano group is used, the biotin derivative (4) can be produced, for example, through a hydrolysis reaction and a de-protection reaction of the biotin derivative (3) by contacting the biotin derivative (3) with a hydrogen halide and a phosgene compound. For example, hydrogen bromide can be used as the hydrogen halide. For example, triphosgene can be used as the phosgene compound.

### EXAMPLES

Hereinafter, the present invention will be described in detail using examples. These examples are merely specific examples, and the present invention is not limited to these examples. Note that, in the Examples and the Comparative Examples, the calculation of the reaction conversion rate and the evaluation of the purity were carried out by the following methods using high performance liquid chromatography (HPLC).

### <HPLC Measurement Conditions>

The analysis conditions for HPLC analysis were as follows.
Device: High performance liquid chromatography (HPLC) device
Model: 2695-2489-2998 (available from Waters Corporation)
Detector: Ultraviolet absorptiometer (measurement wavelength: 210 nm)
Column: XBridge-C18, inner diameter of 4.6 mm, length of 15 cm (particle size: 5 µm) (available from Waters Corporation)
Column temperature: 30°C (constant)
Sample temperature: 25°C (constant)
Mobile phase A: Acetonitrile
Mobile phase B: 0.25% acetic acid aqueous solution
Mobile phase delivery: The concentration gradient was controlled by changing the mixing ratio of the mobile phase A and the mobile phase B as described in Table 1 below.
Flow rate: 0.6 mL/min
Measurement time: 40 minutes

### [Table 1]

**Table 1**

| Time After Injection (min) | Mobile Phase A (vol.%) | Mobile Phase B (vol.%) |
|---|---|---|
| 0 to 10 | 40 | 60 |
| 10 to 40 | 40 -> 100 | 60 -> 0 |

Under the above-described measurement conditions of HPLC, the peak of the thiolactone derivative (5) (R¹, R² = Bn) is confirmed at approximately 20.7 min, the peak of the hydroxybiotin derivative (1A) (R¹, R² = Bn, R³ = -CO₂Et) is confirmed at approximately 25.5 min, the peak of the vinylbiotin derivative (2A) (R¹, R² = Bn, R³ = -CO₂Et) is confirmed at approximately 28.5 min, the peak of the biotin derivative (3A) (R¹, R² = Bn, R³ = -CO₂Et) is confirmed at approximately 28.3 min, and the peak of the biotin precursor (4) (R¹, R² = Bn, R³ = -CO₂H) is confirmed at approximately 19.9 minutes. In the Examples and the Comparative Examples, the purities of the hydroxybiotin derivative (1A), the vinylbiotin derivative (2A), and the biotin derivative (3A) are ratios (percentages) of the peak area values of the hydroxybiotin derivative (1A), the vinylbiotin derivative (2A), and the biotin derivative (3A) to the total of all peak area values (excluding solvent-derived peaks) measured under the above-described conditions, respectively. In addition, the reaction conversion rate is a value calculated as a percentage of the peak area value of the produced biotin derivative (3A) to a total value of the peak area value of the hydroxybiotin derivative (1A) or vinylbiotin derivative (2A) and the peak area value of the biotin derivative (3A). Specifically, when the hydroxybiotin derivative (1A) is used as a raw material, the reaction conversion rate of the biotin derivative (3A) is a value calculated as a percentage of the peak area value of the produced biotin derivative (3A) to a total value of the peak area value of the hydroxybiotin derivative (1A) and the peak area value of the biotin derivative (3A). When the vinylbiotin derivative (2A) is used as a raw material, the reaction conversion rate of the biotin derivative (3A) is a value calculated as a percentage of the peak area value of the produced biotin derivative (3A) to a total value of the peak area value of the vinylbiotin derivative (2A) and the peak area value of the biotin derivative (3A).

### [Production Example 1 (Synthesis of Hydroxybiotin Derivative)]

As shown in the following reaction scheme, a hydroxybiotin derivative (1A) represented by formula (1A) was synthesized from a thiolactone derivative (5) represented by formula (5). In the formula, "Bn" represents a benzyl group, and "Et" represents an ethyl group.

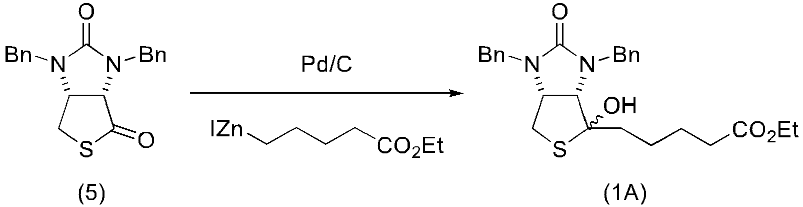

A 100 mL three-neck flask equipped with a stirrer piece having a diameter of 2.5 cm was charged with 6.13 g (93.72 mmol) of zinc powder, 10 mL of tetrahydrofuran, and 7 mL of toluene in a nitrogen atmosphere, and the contents were mixed and stirred at 25°C. Subsequently, 3.12 g of bromine was added dropwise over 2 hours while the temperature was maintained at 30°C or less. The resulting suspension was heated to 55°C, and 10 g (39.05 mmol) of ethyl 5-iodovalerate was added dropwise over 1 hour or more. Next, the mixture was stirred at the same temperature for 2 hours, and then cooled to 30°C, thereby obtaining a solution of a zinc reagent in ethyl 5-iodovalerate.

Subsequently, 8.8 g (26.03 mmol) of the thiolactone derivative (5) and 20 mL of toluene were added to the zinc reagent solution, and the components were mixed and stirred at 30°C. Next, a solution obtained by suspending 277.1 mg (2.60 mmol) of 10 mass% palladium on carbon in 2.6 mL of N,N-dimethylformamide was added to the mixture, and the components were stirred at 40°C for 3 hours. Subsequently, the reaction liquid was cooled to 25°C, and then stirred at the same temperature for 12 hours.

Next, 30 mL of a 5 mass% ammonium chloride aqueous solution was added to the reaction liquid, followed by stirring at 25°C for 30 minutes. The reaction liquid was then filtered, and the filter residue was washed with 20 mL of toluene. The filtered solution was stirred and then allowed to stand, after which the separated aqueous layer was removed. The resulting organic layer was washed with 30 mL of distilled water, 30 mL of a 10 mass% sodium thiosulfate aqueous solution, and 30 mL of a 10 mass% saline solution, respectively. The organic layer was then concentrated, after which 20 mL of toluene was added to the residue, and the mixture was concentrated once again, thereby obtaining 2.0 g of the hydroxybiotin derivative (1A) (purity of 91.71%, and yield of 98.4%).

### [Production Example 2 (Synthesis of Vinylbiotin Derivative)]

As shown in the following scheme, a vinylbiotin derivative (2A) represented by formula (2A) was synthesized from a hydroxybiotin derivative (1A) represented by formula (1A). In the formula, "Bn" represents a benzyl group, and "Et" represents an ethyl group.

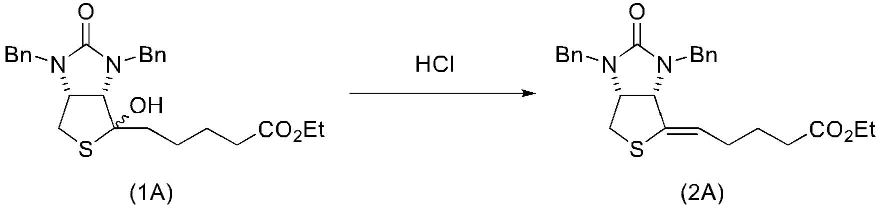

10 g (21.34 mmol) of the hydroxybiotin derivative (1A) obtained in Production Example 1 was weighed in a 100 mL three-neck flask equipped with a stirrer piece having a diameter of 2.5 cm, after which 100 mL of ethanol and 40 mL of an 18 mass% hydrochloric acid aqueous solution were added thereto, followed by stirring at 25°C for 2 hours. After complete disappearance of the raw materials was confirmed by thin layer chromatography (TLC) (with hexane/ethyl acetate = 1:1), the reaction liquid was concentrated. Next, 100 mL of toluene was added for extraction, and the separated water layer was removed. The resulting organic layer was washed with 30 mL of distilled water, 30 mL of a 10 mass% sodium hydrogen carbonate aqueous solution, and 30 mL of a 10 mass% saline solution, respectively. The organic layer was then concentrated, after which 20 mL of toluene was added to the residue, and the mixture was concentrated again, thereby obtaining 9.5 g of the vinylbiotin derivative (2A) (purity of 94.29%, and yield of 99.0%).

### [Production Example 3 (Purification of Vinylbiotin Derivative)]

2.5 g (5.55 mmol) of the vinylbiotin derivative (2A) (purity of 94.29%) obtained in Production Example 2 was purified by column chromatography (hexane/ethyl acetate = 2:1), thereby obtaining 2.1 g of a high-purity vinylbiotin derivative (2A) (purity of 98.82%, and yield of 84.0%).

### [Example 1]

As shown in the following reaction scheme, a biotin derivative (3A) represented by formula (3A) was synthesized from a hydroxybiotin derivative (1A) represented by formula (1A). In the formula, "Bn" represents a benzyl group, and "Et" represents an ethyl group.

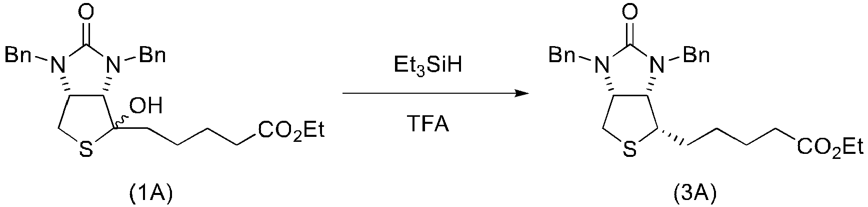

1 g (2.13 mmol) of the hydroxybiotin derivative (1A) (purity of 91.71%) obtained in Production Example 1 was weighed in a 50 mL four-neck flask equipped with a stirrer piece having a diameter of 2.5 cm, after which 5 mL (65.34 mmol) of trifluoroacetic acid (pKa = -0.3, 25°C) was added, and the mixture was cooled to 0°C. Subsequently, 0.74 g (6.40 mmol) of triethylsilane was added at 10°C or less, after which the temperature was raised to 25°C, and the mixture was stirred at the same temperature for 5 hours. The reaction conversion rate of the biotin derivative (3A) after stirring at 25°C for 5 hours was confirmed to be 100% through high performance liquid chromatography (HPLC).

### [Example 2]

As shown in the following reaction scheme, a biotin derivative (3A) represented by formula (3A) was synthesized from a vinylbiotin derivative (2A) represented by formula (2A). In the formula, "Bn" represents a benzyl group, and "Et" represents an ethyl group.

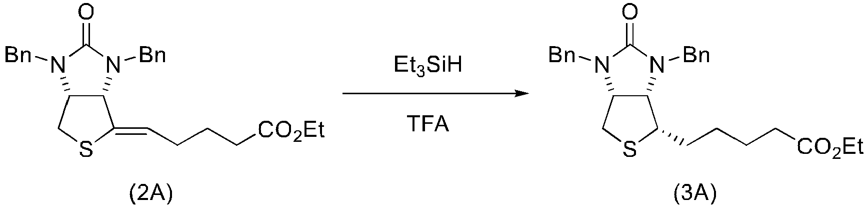

1 g (2.22 mmol) of the vinylbiotin derivative (2A) (purity of 94.29%) obtained in Production Example 2 was weighed in a 50 mL four-neck flask equipped with a stirrer piece having a diameter of 2.5 cm, after which 5 mL (65.34 mmol) of trifluoroacetic acid (pKa = -0.3) was added, and the mixture was cooled to 0°C. Subsequently, 0.77 g (6.66 mmol) of triethylsilane was added at 10°C or less, after which the temperature was raised to 25°C, and the mixture was stirred at the same temperature for 5 hours. The reaction conversion rate of the biotin derivative (3A) after stirring at 25°C for 5 hours was confirmed to be 100% through high performance liquid chromatography (HPLC).

### [Example 3]

A reaction was carried out in the same manner as in Example 2 with the exception that the vinylbiotin derivative (2A) (purity of 98.82%) obtained in Production Example 3 was used. The results are shown in Table 2. The reaction conversion rate of the biotin derivative (3A) after stirring at 25°C for 5 hours was confirmed to be 100%.

### [Example 4]

A reaction was carried out in the same manner as in Example 2 with the exception that the amount of the triethylsilane used was changed to 0.39 g (3.33 mmol). The results are shown in Table 2. The reaction conversion rate of the biotin derivative (3A) after stirring at 25°C for 5 hours was confirmed to be 100%.

### [Example 5]

A reaction was carried out in the same manner as in Example 2 with the exception that the amount of the trifluoroacetic acid used was changed to 1 mL (13.07 mmol). The results are shown in Table 2. The reaction conversion rate of the biotin derivative (3A) after stirring at 25°C for 5 hours was confirmed to be 81.2%. Stirring was continued at 25°C for a total of 24 hours, after which the reaction conversion rate of the biotin derivative (3A) was confirmed to be 100%.

### [Example 6]

A reaction was carried out in the same manner as in Example 2 with the exception that methanesulfonic acid (pKa = -2.6) was used instead of trifluoroacetic acid (pKa = -0.3). The results are shown in Table 2. The reaction conversion rate of the biotin derivative (3A) after stirring at 25°C for 5 hours was confirmed to be 73.3%. Stirring was continued at 25°C for a total of 24 hours, after which the reaction conversion rate of the biotin derivative (3A) was confirmed to be 100%.

### [Examples 7 and 8]

Reactions were carried out in the same manner as in Example 2 with the exception that the amounts of the trifluoroacetic acid used and the triethylsilane used and the reaction temperature were changed as shown in Table 2. The results are shown in Table 2. In Example 7, the reaction conversion rate of the biotin derivative (3A) after stirring at 25°C for 5 hours was confirmed to be 67.2%. Stirring was continued at 25°C for a total of 24 hours, after which the reaction conversion rate of the biotin derivative (3A) was confirmed to be 90.4%. In Example 8, the reaction conversion rate of the biotin derivative (3A) after stirring at 40°C for 5 hours was confirmed to be 87.1%. Stirring was further continued at 40°C for a total of 24 hours, after which the reaction conversion rate of the biotin derivative (3A) was confirmed to be 100%.

### [Comparative Example 1 (Formic Acid)]

1 g (2.22 mmol) of the vinylbiotin derivative (2A) (purity of 98.82%) obtained in Production Example 3 was weighed in a 50 mL four-neck flask equipped with a stirrer piece having a diameter of 2.5 cm, after which 5 mL (131.98 mmol) of formic acid (pKa = 3.55) was added, and the mixture was cooled to 0°C. Subsequently, 0.77 g (6.66 mmol) of triethylsilane was added at 10°C or less, after which the temperature was raised to 25°C, and the mixture was stirred at the same temperature for 5 hours. The reaction conversion rate of the biotin derivative (3A) after stirring at 25°C for 5 hours was confirmed to be 0% through high performance liquid chromatography (HPLC). Stirring was further continued at 25°C for a total of 24 hours, after which the reaction conversion rate of the biotin derivative (3A) was confirmed to be 6.2%.

### [Table 2]

**Table 2**

| | Reaction Substrate | | Strong Acid (Solvent) | | | Trialkylsilane Compound | | Reaction Temperature | Reaction Time | Reaction Conversion Rate |
|---|---|---|---|---|---|---|---|---|---|---|
| | Production Example | Purity (%) | Type | pKa | Amount* | Type | Mole Number** | (°C) | (h) | (%) |
| Example 1 | Production Example 1 | 91.71 | Trifluoroacetic acid | -0.3 | 5 | Triethylsilane | 3.0 | 25 | 5 | 100 |
| Example 2 | Production Example 2 | 94.29 | Trifluoroacetic acid | -0.3 | 5 | Triethylsilane | 3.0 | 25 | 5 | 100 |
| Example 3 | Production Example 3 | 98.82 | Trifluoroacetic acid | -0.3 | 5 | Triethylsilane | 3.0 | 25 | 5 | 100 |
| Example 4 | Production Example 2 | 94.29 | Trifluoroacetic acid | -0.3 | 5 | Triethylsilane | 1.5 | 25 | 5 | 100 |
| Example 5 | Production Example 2 | 94.29 | Trifluoroacetic acid | -0.3 | 1 | Triethylsilane | 3.0 | 25 | 5 | 81.2 |
| | | | | | | | | | 24 | 100 |
| Example 6 | Production Example 2 | 94.29 | Methanesulfonic acid | -2.6 | 5 | Triethylsilane | 3.0 | 25 | 5 | 73.3 |
| | | | | | | | | | 24 | 100 |
| Example 7 | Production Example 2 | 94.29 | Trifluoroacetic acid | -0.3 | 1 | Triethylsilane | 1.5 | 25 | 5 | 67.2 |
| | | | | | | | | | 24 | 90.4 |
| Example 8 | Production Example 2 | 94.29 | Trifluoroacetic acid | -0.3 | 1 | Triethylsilane | 1.5 | 40 | 5 | 87.1 |
| | | | | | | | | | 24 | 100 |
| Comparative Example 1 | Production Example 3 | 98.82 | Formic acid | 3.55 | 5 | Triethylsilane | 3.0 | 25 | 5 | 0 |
| | | | | | | | | | 24 | 6.2 |

| | | | | | | | | | | |
|---|---|---|---|---|---|---|---|---|---|---|
| *Amount used (mL) per 1 g of the reaction substrate (hydroxybiotin derivative or vinylbiotin derivative) **Amount used (mol) per 1 mol of the reaction substrate (hydroxybiotin derivative or vinylbiotin derivative) | | | | | | | | | | |

## Claims

1. A method for producing a biotin derivative, the method comprising contacting, in a solvent containing 40 vol.% or more of a strong acid having an acid dissociation constant pKa of 1 or less,
one or more derivatives selected from the group consisting of:
a hydroxybiotin derivative represented by formula (1): 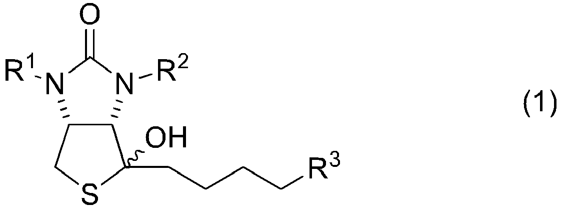 wherein
R¹ and R² each independently represent a hydrogen atom, an alkyl group optionally having a substituent(s), an aralkyl group optionally having a substituent(s), or an aryl group optionally having a substituent(s),
R³ represents a hydrogen atom, an alkyl group optionally having a substituent(s), a cyano group, or a monovalent group represented by the formula: -C(=O)OR⁴, and
R⁴ represents a hydrogen atom, an alkyl group optionally having a substituent(s), an aralkyl group optionally having a substituent(s), or an aryl group optionally having a substituent(s); and
a vinylbiotin derivative represented by formula (2): 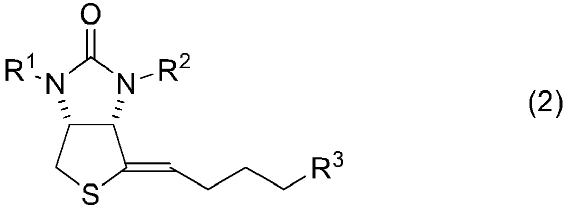 wherein R¹, R² and R³ have the same definitions as R¹, R² and R³ in formula (1), respectively,
with a trialkylsilane compound to produce a biotin derivative represented by formula (3): 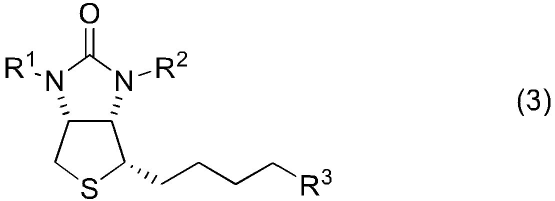 wherein R¹, R² and R³ have the same definitions as R¹, R² and R³ in formula (1), respectively.

2. The method for producing a biotin derivative as claimed in claim 1, wherein the trialkylsilane compound is selected from the group consisting of trimethylsilane, triethylsilane, triisopropylsilane, and trihexylsilane.

3. The method for producing a biotin derivative as claimed in claim 1 or 2, wherein the one or more derivatives each have a purity of 95% or less as measured by liquid chromatography.

4. The method for producing a biotin derivative as claimed in claim 1 or 2, wherein 0.1 mL to 20 mL of the solvent is used per 1 g of the one or more derivatives.

5. The method for producing a biotin derivative as claimed in claim 3, wherein 0.1 mL to 20 mL of the solvent is used per 1 g of the one or more derivatives.
